# EUROPEAN PATENT APPLICATION

(11) **EP 4 129 404 A1**
(43) Date of publication of application: **08.02.2023**
(21) Application number: 21778784.5
(22) Date of filing: 18.03.2021
(51) Int. Cl.: A61P 21/00, A61P 21/06, A61P 43/00, A61K 36/064, A23L 33/10, A23L 33/14, A23L 33/175, A61K 47/36, A61K 31/132, A61K 31/198

(54) **MUSCLE ENHANCER**

(30) Priority: 03.04.2020 JP 2020067732
(71) Applicant: MITSUBISHI GAS CHEMICAL COMPANY, INC., Chiyoda-ku Tokyo 100-8324 (JP)
(72) Inventor: NAKAMURA, Kenji, Niigata-shi, Niigata 950-3112 (JP); FURUTANI, Masayuki, Niigata-shi, Niigata 950-3121 (JP); TACHIKAWA, Tomokazu, Tokyo 100-8324 (JP)
(74) Representative: Vossius & Partner Patentanwälte Rechtsanwälte mbB
(86) International application number: PCT/JP2021/011084
(87) International publication number: WO 2021/200216

(57) **Abstract**

A muscle enhancer containing a polyamine and a branched-chain amino acid as active ingredients.

## Description

### Technical Field

The present invention relates to a muscle enhancer.

### Background Art

For muscle building, that is, increasing and maintaining muscle mass and muscle strength, and further suppressing the decrease in muscle mass and muscle strength, it is said that both appropriate nutritional intake and exercise are required. Then, in view of nutritional intake, it is considered important for muscle building to improve the absorption with respect to the intake of nutrients and to improve the efficiency of biological function to convert absorbed peptides and amino acids into muscle proteins.

Meanwhile, it is generally known that steroids and growth hormones are used as muscle enhancers, and anabolic steroids such as testosterone cypionate and methyltestosterone are used, for example. However, such a muscle enhancer may act on a part other than the muscle and is problematic due to side effects such as an increase in blood pressure/cholesterol level and liver injury at the same time.

Therefore, research and development have also been performed on muscle enhancers with a particular focus on safety, and use of olive extracts or the like has been reported, for example (e.g., Patent Literature 1). In this way, if a muscle enhancer with a focus on safety can be provided, it is considered to be widely used for improving the athletic performance in sports, preventing or improving obesity and metabolic syndrome, and slimming.

### Citation List

### Patent Literature

Patent Literature 1: Japanese Translation of PCT International Application Publication No. 2010-505784

### Summary of Invention

### Technical Problem

The present invention has been devised in view of the problems described above, and an object thereof is to provide a novel muscle enhancer that is expected to improve the absorption with respect to the intake of nutrients and the efficiency of biological function to convert absorbed peptides and amino acids into muscle proteins.

### Solution to Problem

As a result of diligent studies in order to solve the above problems, the inventors have found that the aforementioned problems can be solved by using a polyamine and a branched-chain amino acid in combination, thereby accomplishing the present invention.

That is, the present invention is as follows.
[1] A muscle enhancer comprising a polyamine and a branched-chain amino acid as active ingredients.
[2] The muscle enhancer according to [1], wherein the polyamine is one or more selected from the group consisting of putrescine, spermidine, and spermine.
[3] The muscle enhancer according to [2], comprising 1 mg/g or more of spermidine per 1 g of the muscle enhancer.
[4] The muscle enhancer according to any one of [1] to [3], wherein the polyamine is derived from yeast.
[5] The muscle enhancer according to [4], comprising a polyamine-containing yeast as the polyamine.
[6] The muscle enhancer according to [5], comprising 200 to 800 mg of the polyamine-containing yeast in terms of dry mass per 1 g of the muscle enhancer.
[7] The muscle enhancer according to [5] or [6], comprising 1 mg/g or more of spermidine in terms of dry mass at a moisture content of 6.0% or less.
[8] The muscle enhancer according to any one of [5] to [7], further comprising a crystalline polysaccharide.
[9] The muscle enhancer according to [8], wherein the mass ratio between the yeast and the crystalline polysaccharide is 1:0.1 to 1.
[10] The muscle enhancer according to any one of [4] to [9], wherein the yeast is a yeast belonging to the genus Saccharomyces.
[11] The muscle enhancer according to any one of [1] to [10], wherein the branched-chain amino acid is one or more selected from the group consisting of L-valine, L-leucine, and L-isoleucine.
[12] The muscle enhancer according to [11], comprising 20 to 30 parts by mass of L-valine, 40 to 60 parts by mass of L-leucine, and 20 to 30 parts by mass of L-isoleucine, based on 100 parts by mass in total of L-valine, L-leucine, and L-isoleucine.
[13] The muscle enhancer according to any one of [1] to [12], comprising 200 to 800 mg of the branched-chain amino acid in terms of dry mass per 1 g of the muscle enhancer.
[14] A composition comprising the muscle enhancer according to any one of [1] to [13].
[15] The composition according to [14], being in the form of a food or drink.

### Advantageous Effect of Invention

The present invention can provide a novel muscle enhancer that is expected to improve the absorption with respect to the intake of nutrients and the efficiency of biological function to convert absorbed peptides and amino acids into muscle proteins.

### Description of Embodiments

Hereinafter, the embodiments of the present invention (which will be hereinafter referred to as "the present embodiment(s)") will be described in detail, but the present invention is not limited to these embodiments, and various modifications are possible within the range that does not deviate from the gist.

### [Muscle enhancer]

The muscle enhancer of the present embodiments contains a polyamine and a branched-chain amino acid as active ingredients, and may contain another additive such as a crystalline polysaccharide, as required.

A polyamine, which is a linear in vivo aliphatic hydrocarbon having two or more primary amino groups, has a high effect on cell activation, and foods containing it have been provided. Known physiological actions of polyamine include cell proliferation action, cell differentiation-promoting action, immune essential factor, antiallergic action, protein synthesis-promoting action, structural stabilization by interaction with nucleic acid, and enzyme activity-regulating action. Recently, it has been found that a novel muscle enhancer can be provided by using such a polyamine in combination with a branched-chain amino acid. Hereinafter, each component will be described in detail.

### (Polyamine)

A polyamine is a general term for linear aliphatic hydrocarbons in which multiple amino groups are bonded. Examples thereof include putrescine, spermidine, spermine, 1,3-diamino propane, cadaverine, caldin, homospermidine, aminopropylcadaverine, theremin, thermospermine, canavalmine, aminopentylnorspermidine, N,N-bis(aminopropyl)cadaverine, homospermine, caldopentamine, homocaldopentamine, caldohexamine, and homocaldohexamine spermidine. Among these, putrescine, spermidine, and spermine are preferable. Such a polyamine has a high cell activation ability and a high utilization rate in the body, and is excellent in terms of functionality. In particular, spermidine has a higher acceptable daily intake than spermine and is preferred for higher intakes. Further, use of such a polyamine tends to further improve the muscle-enhancing effect by combination use with the branched-chain amino acid.

The content of polyamine per 1 g of the muscle enhancer is preferably 1 mg/g or more, more preferably 1 to 10 mg/g, further preferably 1 to 5 mg/g. The content of polyamine falling within such a range tends to further improve the muscle-enhancing effect. The content of polyamine is not limited to the aforementioned ranges, and the polyamine can be used within the acceptable daily intake range of the specific compound such as spermidine.

The method for producing a polyamine is not particularly limited. For example, known methods include a preparation method involving treating yeast bacterial cells or yeast culture solutions under acidic conditions, an extraction method involving subjecting plant materials under acidic conditions, a method of producing polyamine extracts by adding a solution of salt such as sodium chloride, magnesium chloride, and calcium chloride to plant materials, or a method for obtaining a polyamine-containing yeast as described in International Publication No. WO 2018/168525.

Among these, the polyamine to be used in the present embodiments is preferably derived from yeast, and it is more preferable to use a polyamine-containing yeast as the polyamine. As the yeast, yeast belonging to the genus Saccharomyces, preferably Saccharomyces cerevisiae, can be used. Use of such a polyamine tends to further improve the muscle-enhancing effect. In this case, the polyamine to be contained can be 1.5 mg or more, preferably 2.0 mg or more, more preferably 2.5 mg or more, per 1 g (dry mass) of yeast.

The "dry mass" in the present embodiments means the mass at a moisture content of 6.0% or less, unless otherwise specified.

The dry yeast containing a polyamine can be obtained by removing bacterial cells from a medium containing yeast by filtration or centrifugation, followed by drying. In order to remove the medium components, washing may be performed. For drying the yeast, spray drying, vacuum drying, freeze drying, and the like can be used, and those skilled in the art can appropriately select a drying method according to the purpose.

The polyamine to be contained in the muscle enhancer may be added in the form of a compound of the polyamine or may be added in the form of a polyamine-containing yeast, as mentioned above. Among these, addition in the form of a polyamine-containing yeast is preferable.

In the case of adding a polyamine in the form of a polyamine-containing yeast to the muscle enhancer, the dry mass thereof is preferably 200 to 800 mg, more preferably 300 to 700 mg, further preferably 400 to 600 mg, per 1 g of the muscle enhancer. The dry mass of the polyamine-containing yeast falling within such a range tends to further improve the muscle-enhancing effect. The content of polyamine is not limited to the aforementioned ranges, and it can be used within the acceptable daily intake range of the specific compound such as spermidine.

In the case of addition in the form of a polyamine-containing yeast, the content of spermidine is preferably 0.8 mg/g or more, more preferably 0.9 mg/g or more, further preferably 1 mg/g or more, in terms of dry mass at a moisture content of 6.0% or less, per 1 g of the muscle enhancer. The dry mass of polyamine-containing yeast falling within such a range tends to further improve the muscle-enhancing effect. The upper limit of the content of spermidine in the case of addition in the form of a polyamine-containing yeast is not particularly limited but is preferably 10 mg/g or less, more preferably 5 mg/g or less.

### (Branched-chain amino acid)

A branched-chain amino acid (BCAA) generally refers to one or more selected from the group consisting of L-valine, L-leucine, and L-isoleucine among nine essential amino acids that cannot be synthesized in the human body and is an important nutrient serving as an energy source for muscles. Branched-chain amino acids are abundantly contained in meat, fish, dairy products, eggs, etc., but since they are ingested as proteins from foods, it takes several hours before they are decomposed into branched-chain amino acids and absorbed. Therefore, in order to efficiently absorb branched-chain amino acids, it is effective to ingest the branched chain amino acids as they are.

The muscle enhancer of the present embodiments preferably contains L-valine, L-leucine, and L-isoleucine as branched-chain amino acids, respectively. In this case, regarding the mass ratio between L-valine, L-leucine, and L-isoleucine, 20 to 30 parts by mass of L-valine, 40 to 60 parts by mass of L-leucine, and 20 to 30 parts by mass of L-isoleucine, based on 100 parts by mass in total of L-valine, L-leucine, and L-isoleucine are preferably contained. The branched-chain amino acids contained at such a mass ratio tend to further improve the muscle-enhancing effect.

The content of branched-chain amino acids in the muscle enhancer of the present embodiments is preferably 200 to 800 mg, more preferably 300 to 700 mg, further preferably 400 to 600 mg, in terms of dry mass, per 1 g of the muscle enhancer. The content of branched-chain amino acids falling within such a range tends to further improve the muscle-enhancing effect.

The method for producing branched-chain amino acids is not specifically limited, and they can be produced by the fermentation method, the synthesis method, the purification method, or the like. Commercially available products also can be used. As the branched-chain amino acids used in the present invention, common commercially available branched-chain amino acids can be used.

### (Crystalline polysaccharide)

The muscle enhancer of the present embodiments may contain a crystalline polysaccharide. Containing a crystalline polysaccharide can impart fluidity or deodorization to the muscle enhancer and also can impart stability to the polyamine or polyamine-containing yeast. Further, it is also possible to suppress fat absorption and contribute to the muscle-enhancing effect.

The crystalline polysaccharide is not particularly limited, and examples thereof include polysaccharides that exist as naturally crystalline solids such as starch, dextrin, glycogen, and cellulose. Among these, cyclodextrin, which is a dextrin having a cyclic structure, can be suitably used as a crystalline polysaccharide. There are alpha, beta, and gamma types of cyclodextrin, all of which can be used as crystalline polysaccharides. Among these, alpha cyclodextrin and gamma cyclodextrin are preferable. Other dextrins such as resistant dextrins can also be used for the composition of the present invention.

It is possible to stabilize the yeast to be easily handled by combining a polyamine-rich yeast with a crystalline polysaccharide. The yeast and the crystalline polysaccharide may be mixed by any method of dry mixing and wet mixing. In the case of wet mixing, the saccharide after being sterilized and mixed with the yeast can be dried. Alternatively, the yeast may be mixed as undried yeast and then dried, instead of being mixed as dried yeast. Further, yeast after being cultured may be collected/concentrated by centrifugation, a crystalline polysaccharide may be added thereto, and then the yeast and the crystalline polysaccharide may be mixed by spray drying.

The content of crystalline polysaccharide is preferably 30 to 500 mg, more preferably 40 to 400 mg, further preferably 50 to 300 mg, per 1 g of the muscle enhancer. The content of crystalline polysaccharide falling within such a range tends to further.improve the muscle-enhancing effect.

In the case of using a polyamine-containing yeast, the mass ratio between the dry mass of the polyamine-containing yeast and the crystalline polysaccharide is preferably 1:0.1 to 3, more preferably 1:0.1 to 2, further preferably 1:0.1 to 1. The mass ratio between the dry mass of the polyamine-containing yeast and the crystalline polysaccharide falling within such a range not only further improves the stability of the polyamine-containing yeast, but also can ensure the content of polyamine, especially spermidine in the muscle enhancer to a certain extent, so that the effect of the muscle enhancer can be obtained with an appropriate intake.

### [Composition]

The composition of the present embodiment contains the muscle enhancer described above. The application thereof is not particularly limited, and it can be used, for example, for not only various general foods or drinks, but also foods or drinks, pharmaceutical products, non-medicinal products, and cosmetics that are labeled with functionality so that they can be ingested by individuals who needs nutrient enrichment, especially, individuals who needs cell activity.

Such foods or drinks are not particularly limited, and examples thereof include specified health foods or drinks, nutritional functional foods or drinks, functional epidermal foods or drinks, health functional foods or drinks, special purpose foods or drinks, nutritional supplements, health supplements, supplements, beauty foods or drinks, and other health foods or drinks.

### Examples

Hereinafter, the present invention will be described more specifically with reference to examples and comparative examples. The present invention is not limited by the following examples at all.

### (Preparation of test foods)

### (Capsules A)

A BCAA agent as a branched-chain amino acid was prepared at a mass ratio of L-valine:L-leucine:L-isoleucine of 1:2:1. 123 mg of Elion SP (R), available from MITSUBISHI GAS CHEMICAL COMPANY, INC., which is a yeast food containing spermidine, and 112 mg of the BCAA agent prepared above were mixed and put into hard capsules No. 2, to prepare capsules A. The dry mass of the spermidine-containing yeast in Elion SP (R) was adjusted to 100 mg. 3 mg of spermidine was contained per 1 g of the dry mass of the spermidine-containing yeast.

### (Capsules B)

123 mg of corn starch and 112 mg of the BCAA agent prepared above were mixed and put into hard capsules No. 2, to prepare capsules B for placebo.

### (Capsules C)

235 mg of corn starch was put into hard capsules No. 2, to prepare capsules C for placebo.

### (Grouping of subjects and daily intake)

Subjects satisfying the following conditions were selected and subjected to the following tests.
· Healthy men over 50 years old (50s, 60s, and 70s) who have not been treated for illness
· Those with a BMI value of 18.5 or more and less than 30
· Those who have not taken supplements (BCAAs, proteins, etc.) that may affect muscles within one month before the start of the test
· Those who have not taken Natto, which contains a lot of polyamines, more than twice a week

A pre-screening test (before ingestion) was performed on the aforementioned subjects to determine suitability. Then, the subjects were classified into the following Groups I, II, and III based on the daily capsule intake.
Group I: Group taking 3 capsules A and 6 capsules B per day, with a yeast intake of 300 mg/day and a BCAA agent intake of 1008 mg/day
Group II: Group taking 9 capsules B per day, with a yeast intake of 0 mg/day and a BCAA agent intake of 1008 mg/day
Group III: Group taking 9 capsules C per day, with a yeast intake of 0 mg/day and a BCAA agent intake of 0 mg/day

BCAA agents have already been commercially available from many manufacturers, and most products recommend an intake of approximately 2000 mg or more per day. However, in order to determine the effect of polyamine, the intake of the BCAA agent in this evaluation was set to 1008 mg (about 1/2 of the general recommended intake).

### (Test method)

Human oral clinical trials were conducted in a double-blind study on subjects in Groups I, II, and III. Specifically, during the test period, each subject took a total of 9 capsules within 30 minutes after each breakfast, and this test was conducted for 8 weeks. During the test period, the subject was asked to keep a daily step count, exercise, and dietary records as a guideline for exercise indicators. After that, the subject was inspected for the same items as in the pre-examination.

### (Inspection items)

Before and after the test, the subject was inspected for muscle mass and SMI with a body composition analyzer as a physiological test in addition to a blood test. The definition of each term is shown below (see the instruction manual of TANITA CORPORATION). The body composition analyzer used is MC-980A-N plus, available from TANITA CORPORATION. This body composition analyzer is a body composition analyzer using the bioelectrical impedance method.
Muscle mass: The mass of muscle tissue (skeletal muscle, smooth muscle, water content) that maintains posture and moves the heart, excluding fat mass and estimated bone mass.
SMI: Abbreviation for Skeletal Muscle Mass Index.
Total value of left arm, right arm, left leg, right leg muscle mass (kg)/height² (m)

### (Analysis)

The difference in each item before and after the test measured as described above was evaluated by the Wilcoxon rank sum test among the nonparametric tests. In this evaluation, when the p-value is p < 0.05 (*), it was determined as being statistically significant, and when the p-value is p < 0.01 (**), it was determined as being highly significant.

The correlation coefficient (r) between the intake of Elion SP (R) and the difference before and after the test was determined in Groups I, II, and III. In this evaluation, when the r value is 0.4 ≤ | r | ≤ 0.7, it is regarded as "correlated (*)", and when the r value is 0.7 < | r | < 1.0, it is "highly correlated (**)".

### (Results 1)

Table 1 shows the results when the aforementioned analysis was performed on the subjects belonging to each of Group I and Group II. In a nonparametric test, no significant difference was found between the intake of Elion SP (R) and the items related to the muscle mass in Group I. However, in correlation coefficient, a correlation was found between the intake of Elion SP (R) and the muscle mass.

**[Table 1]**

| | n number | Average number of steps | Muscle mass | SMI |
|---|---|---|---|---|
| GROUP II (Average value) | 7 | 9315 | 0.257 | 0.111 |
| GROUP I (Average value) | 10 | 8303 | 0.970 | 0.309 |
| GROUP I (p value) | | | p≥0.05 | p≥0.0 5 |
| Correlation coefficient (r) | - | - | 0.516* | 0.376 |

### (Results 2)

Table 2 shows the results of performing statistical calculations again based on Group III, with the difference before and after the test taken as 0. As a result, in a nonparametric test, significant differences were found between the intake of Elion SP (R) and the muscle mass and SMI in Group I, and no significant difference was found between the intake of Elion SP (R) and the items related to the muscle mass in Group II.

**[Table 2]**

| | n number | Muscle mass | SMI |
|---|---|---|---|
| GROUP III | 10 | - | - |
| GROUP II | 7 | p≥0.05 | p≥0.05 |
| GROUP I | 10 | p<0.01** | p<0.01** |

### (Results 3)

Table 3 shows the results when the subjects belonging to each of Group I and Group II were further divided into "exercisers" and "non-exercisers", and the aforementioned analysis was performed on "exercisers".

The "exercisers" were defined as those with an average number of steps per day equal to or higher than the literature value of the average number of steps in each age group, including the second quartile borders when divided by quartiles. The literature was cited from "Table 61 of the FY2017 National Nutrition Survey" published on the website of the Ministry of Health, Labour and Welfare.

From these results, significant differences were found in muscle mass and SMI in Group I, and correlations were also found in muscle mass and SMI in correlation coefficient.

**[Table 3]**

| | n number | Average number of steps | Muscle mass | SMI |
|---|---|---|---|---|
| GROUP II (Average value) | 4 | 11181 | -0.400 | -0.085 |
| GROUP I (Average value) | 5 | 10597 | 1.160 | 0.382 |
| GROUP I (p value) | | | p<0.05* | p<0.0 5* |
| Correlation coefficient (r) | - | - | 0.560* | 0.448* |

### Industrial Applicability

The present invention has industrial applicability in that it provides a novel muscle enhancer.

## Claims

1. A muscle enhancer comprising:
a polyamine and a branched-chain amino acid as active ingredients.

2. The muscle enhancer according to claim 1, wherein
the polyamine is one or more selected from the group consisting of putrescine, spermidine, and spermine.

3. The muscle enhancer according to claim 2, comprising:
1 mg/g or more of spermidine per 1 g of the muscle enhancer.

4. The muscle enhancer according to any one of claims 1 to 3, wherein
the polyamine is derived from yeast.

5. The muscle enhancer according to claim 4, comprising:
a polyamine-containing yeast as the polyamine.

6. The muscle enhancer according to claim 5, comprising:
200 to 800 mg of the polyamine-containing yeast in terms of dry mass per 1 g of the muscle enhancer.

7. The muscle enhancer according to claim 5 or 6, comprising:
1 mg/g or more of spermidine in terms of dry mass at a moisture content of 6.0% or less.

8. The muscle enhancer according to any one of claims 5 to 7, further comprising:
a crystalline polysaccharide.

9. The muscle enhancer according to claim 8, wherein
the mass ratio between the yeast and the crystalline polysaccharide is 1:0.1 to 1.

10. The muscle enhancer according to any one of claims 4 to 9, wherein
the yeast is a yeast belonging to the genus Saccharomyces.

11. The muscle enhancer according to any one of claims 1 to 10, wherein
the branched-chain amino acid is one or more selected from the group consisting of L-valine, L-leucine, and L-isoleucine.

12. The muscle enhancer according to claim 11, comprising:
20 to 30 parts by mass of L-valine, 40 to 60 parts by mass of L-leucine, and 20 to 30 parts by mass of L-isoleucine, based on 100 parts by mass in total of L-valine, L-leucine, and L-isoleucine.

13. The muscle enhancer according to any one of claims 1 to 12, comprising:
200 to 800 mg of the branched-chain amino acid in terms of dry mass per 1 g of the muscle enhancer.

14. A composition comprising:
the muscle enhancer according to any one of claims 1 to 13.

15. The composition according to claim 14, being in the form of a food or drink.
